# EUROPEAN PATENT APPLICATION

(11) **EP 3 170 900 A1**
(43) Date of publication of application: **24.05.2017**
(21) Application number: 16199758.0
(22) Date of filing: 21.11.2016
(51) Int. Cl.: C12P 19/00, C12P 19/02, C12P 7/10

(54) **A PROCESS FOR CONVERSION OF BIOMASS TO ORGANIC COMPOUND(S)**

(30) Priority: 19.11.2015 IN 4355MU2015
(71) Applicant: Reliance Industries Limited, Mumbai 400 021 Maharashtra (IN)
(72) Inventor: Sankh, Santoshkumar, 416413 Dist: Sangli (IN); Phadke, Makarand, 411008 Pashan Pune (IN); Sarangan, Swaroop, 41026 Navi Mumbai (IN); Rangaswamy, Vidhya, 400605 Kalwa Thane (IN)
(74) Representative: HGF Limited

(57) **Abstract**

The present disclosure relates to the field of organic chemistry in general. Particularly, the present invention relates to a process for conversion of biomass to simpler organic compound(s) such as carbohydrates. The process of the present disclosure provides for pretreatment of biomass using acids organic acids and/or inorganic acids and further treatment using de-esterification agent, thereby rendering the biomass more amenable to enzymatic hydrolysis. The present disclosure also relates to application of the process in biofuel production by fermenting the organic products obtained by the process of the disclosure.

## Description

### TECHNICAL FIELD

The present disclosure relates to field of organic chemistry in general. Particularly, the present disclosure relates to a process for conversion of biomass to simpler organic compound(s) such as carbohydrates. More particularly, the present disclosure provides for pre-treatment of biomass by employing acids, not limiting to organic acids and/or inorganic acids and further treatment using de-esterification agent, thereby rendering the biomass more amenable to enzymatic hydrolysis. The present disclosure also relates to application of the process in biofuel production by fermenting the organic products obtained by the process of the disclosure.

### BACKGROUND

Any discussion of the prior art throughout the specification should in no way be considered as an admission that such prior art is widely known or forms part of common general knowledge in the field.

Biomaterials such as biomass, residues/waste of various natural and man-made activities such as industrial wastes and effluents etc. are abundantly available. Biomass is a renewable energy resource derived from the waste obtained after performing various activities and from numerous sources, including agricultural residues or agricultural wastes, by-products from industries such as timber industry, agricultural crops, raw material from the forests/woods, household wastes and wood etc. The biomass among various other components also includes various complex carbohydrates such as hemicellulose, cellulose (both being carbohydrate polymers) and lignin etc. Sugars obtained from hemicellulose and cellulose, which are part of the biomass are useful for production of variety of industrial chemicals via biological or chemical route.

Agricultural biomass/Energy crops such as cotton stalk, castor stalk, red gram etc are abundant in India. Therefore, these biomass feedstock's can be used as a source of carbon for various second generation biofuels such as bioethanol, biobutanol and high value chemicals such as furfural, HMF, levulinic acid etc. Growing concerns over the environmental impact of fossil fuels and their inevitable depletion have led to intense research on the development of alternative energy sources that can reduce the dependence on foreign oil imports.

Presently, corn, sugarcane molasses are the primary raw material for ethanol production. Using lignocellulosic feedstocks, ethanol can be produced at a lower cost as compared to corn. Development of technology for conversion of lignocellulosic biomass/feedstock to ethanol is complex and challenging than conversion of corn or sugarcane molasses due to the necessity to break the rigid structure of plant. Pre-treatment is required to alter the structural and chemical composition of lignocellulosic biomass to facilitate rapid and efficient hydrolysis of carbohydrates to fermentable sugars. To use all these biomass for bio refinery, it needs to be fractionated into major constituents such as cellulose, hemicellulose and lignin.

Cotton stalk is an agricultural residue rich in cellulose (32-46%) and hemicellulose (20-28%) which is currently not being used as feed or fuel. One promising technology is to convert this abundant and renewable lignocellulosic biomass to ethanol through an enzyme-based process. However, pre-treatment is one of the most expensive and least technologically developed steps in the process of converting biomass to fermentable sugars. Costs are due to the use of chemicals and the need for expensive corrosion-resistant reactors. An efficient pre-treatment method must be developed to disrupt the recalcitrant structure of lignocellulosic biomass and increase the cellulose available for enzymatic hydrolysis ultimately yielding more sugars for fermentation.

A variety of physical, chemical, physico-chemical, and biological pre-treatment techniques have been developed to improve the accessibility of enzymes to cellulosic fibers. Acid pre-treatment involves removal of hemicellulose components and exposing cellulose for enzymatic digestion. Some of the prior arts studied formic acid pre-treatment process. wherein, liquor and washings of formic acid treatment process, rich in hemicellulose (about 58%), also contained considerable amounts of minerals, proteins and organic acids. Utilization of hemicellulose fraction for fermentation is one of the challenges to make process economical as hemicellulose rich liquor is with other sugar degraded components such as furfural, hydroxymethylfurfural, levulinic acid and other phenolics. The existing art also provides for biomass treatment with nitric acid to yield pure cellulose. However, liquor and washings of said acid treatment contain lignin and C5 fractions which cannot be separated.

The present disclosure overcomes the aforementioned limitations observed in the prior art by presenting a method for conversion/deconstruction of biological material including but not limiting to biomass to simpler carbohydrates.

It is an object of the present disclosure to overcome or ameliorate at least one of the disadvantages of the prior art, and/or to provide a useful alternative.

### BRIEF DESCRIPTION OF THE ACCOMPANYING FIGURES

In order that the disclosure may be readily understood and put into practical effect, reference will now be made to exemplary embodiments as illustrated with reference to the accompanying figures. The figures together with a detailed description below, are incorporated in and form part of the specification, and serve to further illustrate the embodiments and explain various principles and advantages, in accordance with the present disclosure wherein:
**Figure 1** illustrates the general process of the present disclosure in the form of a flowchart.
**Figure 2** illustrates the reactor employed for carrying out the pre-treatment step of the instant disclosure. Showcased is a 1L reactor with flush bottom valve; Manufacturer: Amar Equipments Pvt Ltd; Pressure limit: 100 bar, Temperature limit: 350°C and all dimensions of the reactor are provided in mm.
**Figure 3** illustrates the chromatogram depicting xylose in biomass liquor.
**Figure 4** illustrates the chromatogram depicting glucose obtained after enzymatic hydrolysis.

### SUMMARY OF THE DISCLOSURE

The present disclosure relates to a process for conversion of biomass to simpler organic compound(s). In a non-limiting embodiment of the present disclosure, the biomass includes but is not restricted to castor stalk, cotton stalk, red gram stalk, sesame stalk, sugarcane top, blackgram stalk, wheat straw and groundnut stalk or any combinations thereof.

In an exemplary embodiment of the present disclosure, the organic matter present within the biomass is converted to simpler organic compounds such as carbohydrates.

In another exemplary embodiment of the present disclosure, the conversion is by breakdown, deconstruction and hydrolysis or any combinations thereof, of the biomass.

In yet another exemplary embodiment of the present disclosure, the process for conversion of biomass to simpler organic compounds includes but is not limited to pre-treating the biomass using organic acids and/or inorganic acids, thereafter treatment using de-esterification agent, followed by enzymatic hydrolysis.

The present disclosure also relates to organic compounds/simple carbohydrates obtained by employing the process as aforementioned.

The present disclosure also relates to use/application of said process in obtaining simple organic compounds which can be further fermented and employed for biofuel production.

### DETAILED DESCRIPTION OF THE DISCLOSURE

To overcome the non-limited drawbacks of the prior art and to provide for a simple, cost-effective and efficient method for conversion of biomass, the present disclosure relates to a method for treating biomass for rendering the biomass more amenable to the action of hydrolytic enzymes, to yield higher recovery of sugars.

The present disclosure relates to a process for conversion of biomass to organic compound(s) comprising acts of:
pre-treating the biomass with organic acid or inorganic acid or both;
subjecting the pre-treated biomass to de-esterification; and
subjecting the de-esterified biomass to enzyme hydrolysis, thereby converting the biomass to the organic compound(s).

In an embodiment, the biomass is selected from a group comprising red gram stalk, cotton stalk, castor stalk, black gram stalk, sugarcane top, sesame stalk, wheat straw and groundnut stalk or any combination thereof; wherein said biomass comprises complex carbohydrates

In another embodiment, the organic compound(s) is sugar(s), which is selected from a group comprising glucose, xylose, hexose, pentose and arabinose or any combination thereof.

In yet another embodiment, the biomass is subjected to shredding or grinding before pre-treatment; and wherein the biomass is in concentration ranging from about 10% w/v to about 20% w/v.

In still another embodiment, the pre-treating comprises the biomass to be subjected to a step of organic acid treatment, followed by inorganic acid treatment; and wherein the process enhances the conversion of biomass to sugar(s) by at least 50%, when compared to a process devoid of pre-treatment with inorganic acid.

In still another embodiment, the organic acid pre-treatment comprises contacting the biomass with organic acid selected from a group comprising formic acid, acetic acid or a combination thereof; and wherein the organic acid is, in concentration ranging from about 50% w/v to about 90% w/v.

In still another embodiment, the organic acid treated biomass is further subjected to washing and filtering to obtain organic acid pre-treated biomass in the form of pulp and liquor; and wherein the pulp obtained is further subjected to wash treatment followed by drying.

In still another embodiment, the inorganic acid pre-treatment comprises contacting the biomass pulp obtained after the organic acid treatment with the inorganic acid selected from a group comprising nitric acid, sulphuric acid and hydrochloric acid or any combination thereof; and wherein the inorganic acid in concentration ranging from about 0.1% (w/w) to about 5% (w/w).

In still another embodiment, the inorganic acid treated biomass is further subjected to washing and filtering to obtain pre-treated biomass in the form of pulp and liquor; and wherein the pulp obtained is further subjected to wash treatment followed by drying.

In still another embodiment, the pre-treatment is carried out in a reactor, at a temperature ranging from about 100°C to about 180°C for a time-period ranging from about 10 min to about 60 minutes with agitation speed of about 50 rpm to about 500 rpm.

In still another embodiment, ratio of the organic acid to the biomass ranges from about 4:1 to about 10:1, and wherein ratio of the inorganic acid to the biomass ranges from about 0.2:1 to about 1:1.

In still another embodiment, the de-esterification is carried out by subjecting the pre-treated biomass pulp to alkali treatment, wherein the alkali is selected from a group comprising sodium carbonate, sodium hydroxide, potassium hydroxide, calcium hydroxide, calcium carbonate, calcium oxide and ammonia or any combination thereof; which is in concentration ranging from about 0.005% (w/v) to about 1% (w/v)and wherein the ratio of alkali : biomass ranges from about 1:1 to about 1:15 or the alkali is added to the pre-treated biomass pulp until a pH of 11 is obtained.

In still another embodiment, the enzyme hydrolysis is carried out by subjecting the de-esterified biomass to enzyme treatment, wherein the enzyme is a mixture of cellulase, xylanase and beta-glucosidase at a concentration ranging from about 0.05FPU/gram to about 100FPU/gram of biomass.

In still another embodiment, the enzyme hydrolysis is carried out at a pH ranging from about 4 to about 5; incubation temperature ranging from about 40°C to about 60°C; agitation speed ranging from 150 rpm to 250 rpm; and enzyme load of about 25FPU/g to about 100 FPU/g for about 24 hours to about 48 hours.

The present disclosure provides for a method/process of conversion of biomass to simpler organic compounds such as carbohydrates.

In an exemplary embodiment, biomass is subjected to composition analysis.

In an embodiment, composition analysis refers to quantification of cellulose, hemicellulose and lignin in the biomass.

In an exemplary embodiment, conversion of the biomass is by breakdown, deconstruction and hydrolysis or any combination of steps thereof.

In another exemplary embodiment, the breakdown step of biomass treatment involves steps not limiting to grinding, shredding, pre-treatment and/or de-esterification of the biomass.

In a non-limiting embodiment, the present disclosure includes conversion of various biomass, from sources such as but not restricting to biomaterial, residues/waste from various natural as well as man-made activities, industrial wastes and effluents etc. to simpler organic compounds such as carbohydrates.

In an exemplary embodiment, the present disclosure relates to conversion of biomass which includes but is not limited to wastes from living matter or just living matter, wastes derived from various activities including agriculture, agricultural residues, by-products from various industries such as timber industry, agricultural crops, raw material from the forests/woods, household waste and wood etc. In a non-limiting embodiment, the biomass includes but is not restricted to the organic matter present within the biomass.

In another exemplary embodiment, the biomass includes but is not limited to various complex carbohydrates such as hemicellulose, cellulose, lignin etc.

In yet another exemplary embodiment, the biomass is recalcitrant biomass which includes but is not limited to, ligno-cellulosic biomass.

In a non-limiting embodiment, the ligno-cellulose biomass includes but is not restricted to virgin biomass, waste biomass, agricultural biomass/energy crops etc. or any combination thereof.

In another non-limiting embodiment, the agricultural biomass/energy crop includes but is not restricted to castor stalk, cotton stalk, red gram stalk, sesame stalk, sugarcane top, blackgram stalk, wheat straw and groundnut stalk or any combination thereof.

In yet another non-limiting embodiment, the method of conversion of biomass comprises the act of, shredding, pre-treatment, de-esterification and hydrolysis.

In still another non-limiting embodiment, the method of conversion of the biomass to sugars includes but is not limited to the steps of shredding the biomass, pre-treatment of biomass using acids: organic acids and/or inorganic acids, further treatment using de-esterification agent, and thereafter enzymatic treatment.

In an exemplary embodiment, the shredding of the biomass is an optional step.

In a preferred embodiment, the shredding of the biomass is carried out in a shredder to a size ranging from about 0.5mm to about 5mm, more preferably about 2mm.

In an exemplary embodiment, the pre-treatment involves exposure/contacting of the shredded or non-shredded biomass to organic acid(s)/organic acid treatment followed by inorganic acid(s)/ inorganic acid treatment.

In still another non-limiting embodiment, the method of conversion of biomass to obtain simpler organic compounds, comprises the act(s)/step(s) of:
a) obtaining the biomass and optionally subjecting it to shredding;
b) pre-treating the biomass by exposing/contacting the biomass to organic acid treatment followed by inorganic acid treatment, to obtain pre-treated biomass;
c) de-esterifying the pre-treated biomass by adding de-esterification agent to obtain the de-esterified biomass; and
d) subjecting the de-esterified biomass to enzymatic treatment to obtain simpler organic compounds from the biomass.

In still another non-limiting embodiment, the method of conversion of biomass to obtain simpler organic compounds, comprises the act(s)/step(s) of:
a) pre-treating the biomass by exposing/contacting the biomass to organic acid treatment followed by inorganic acid treatment, to obtain pre-treated biomass;
b) de-esterifying the pre-treated biomass by adding de-esterification agent to obtain the de-esterified biomass; and
c) subjecting the de-esterified biomass to enzymatic treatment to obtain simpler organic compounds from the biomass.

In still another non-limiting embodiment, the method of conversion of biomass to obtain simpler organic compounds, comprises the act(s)/step(s) of:
a) pre-treating the biomass by exposing/contacting the biomass to organic acid treatment optionally followed by inorganic acid treatment, to obtain pre-treated biomass;
b) de-esterifying the pre-treated biomass by adding de-esterification agent to obtain the de-esterified biomass; and
c) subjecting the de-esterified biomass to enzymatic treatment to obtain simpler organic compounds from the biomass.

In still another non-limiting embodiment, the organic acid treatment is carried out by adding to the biomass, organic acid such as but not limited to acetic acid or formic acid.

In a preferred embodiment, the organic acid is formic acid.

In still another non-limiting embodiment, the organic acid treatment is carried out by adding organic acid not limiting to formic acid to the biomass, followed by inorganic acid treatment.

In a preferred embodiment, the organic acid treatment is carried out by adding formic acid, followed by inorganic acid treatment.

In still another non-limiting embodiment, the inorganic acid is a strong acid, wherein the strong acid is selected from a group comprising, but not restricted to sulphuric acid, hydrochloric acid and nitric acid or a combination thereof.

In a preferred embodiment, the inorganic acid is a strong acid such as nitric acid.

In another preferred embodiment, the organic acid treated biomass is subjected to inorganic acid treatment by employing nitric acid. Said inorganic acid treatment step is also alternatively referred to as nitration.

In an exemplary embodiment, the ratio of formic acid: biomass is at about 4:1 to about 10:1.

In a preferred embodiment, the ratio of formic acid: biomass is about 4:1.

In another exemplary embodiment, the ratio of nitric acid: biomass is at about 0.2:1 to about 1:1.

In a preferred embodiment, the ratio of nitric acid: biomass is about 0.4:1.

In a non-limiting embodiment, the pre-treatment is carried out at temperature ranging from about 100°C to about 180°C, preferably about 130°C for period of time ranging from about 10 min to about 60 minutes, preferably about 30 minutes with agitation speed of about 50 rpm to about 500 rpm, preferably about 200 rpm-300 rpm.

In an exemplary embodiment, the biomass employed in the instant disclosure is in the concentration ranging from about 10% w/v to about 20% w/v, preferably about 16.6% w/v; formic acid is in the concentration ranging from about 50% w/v to about 90% w/v, preferably about 67% w/v; and nitric acid is in the concentration ranging from about 0.1% w/w to about 5%w/w, preferably about 1% w/w to 2%w/w.

In another exemplary embodiment, the pre-treatment of the biomass is carried out in a system such as but not limiting to a reactor, preferably a stirred-tank reactor.

In yet another exemplary embodiment, the pressure in the reactor goes up to about 3-4 bar because of the rising temperature in the reactor.

In yet another exemplary embodiment, after organic acid pre-treatment, the whole slurry from the reactor is cooled to a temperature ranging from about 50-60°C.

In still another exemplary embodiment, the cooled slurry is filtered.

In an embodiment, filtration removes all insoluble biomass particles.

In a non-limiting embodiment, the slurry is filtered through 297 µm mesh followed by 37 µm mesh, to obtain pulp and liquor.

In another non-limiting embodiment, in the pre-treatment step, the biomass obtained after organic acid treatment and filtration yields pulp and liquor.

In an embodiment, the pre-treatment with an organic acid like formic acid results in release of C5 compounds (from the hemicellulose breakdown to release xylose) in the liquor (the liquor here contains purely C5 compounds with negligible amount of lignin) with lignin and cellulose intact in the pulp (biomass). The consequent treatment of the organic acid pre-treated pulp with inorganic acid, like nitric acid, results in delignification and thus release of lignin monomers into the liquor (the liquor here contains purely lignin with negligible amounts of glucose and xylose) and cellulose remaining intact in the pulp (biomass). Reversing the order of these steps would play out in the following manner. Nitric acid treatment will only result in cellulose remaining intact in the biomass pulp with the liquor containing both lignin and C5 carbons (separating C5 sugars and lignin monomers from a single liquor stream is a difficult recovery process. Obtaining separate streams of lignin and C5 sugars is beneficial as in subsequent step of fermentation, lignin hinders the microbial intake of C5 sugars. Getting a pure C5 stream is therefore the objective and benefit of our disclosure).

In an embodiment, the boiling point of formic acid at a range of about 100°C makes it easier to remove it and reuse it simultaneously making it easier to separate the xylose from the same product stream.

In another non-limiting embodiment, the pulp obtained after organic acid pre-treatment is subjected to water wash or washing, wherein, the washings obtained after the water wash contains glucose and xylose.

In a preferred embodiment, two water washings are performed.

In another preferred embodiment, glucose and xylose in pulp washing along with liquor are analyzed by HPLC.

In yet another non-limiting embodiment, the pulp washing contains glucose and xylose and the liquor contains C5 sugars.

In a preferred embodiment, the pulp is dried and dried pulp is subjected to inorganic acid treatment or nitration to obtain pre-treated biomass; and the liquor is stored for further analysis.

In a preferred embodiment, xylose in the liquor/biomass liquor is analyzed by HPLC.

In another preferred embodiment, xylose in biomass liquor has a purity of about 64.59%.

In an embodiment, pulp is dried at a temperature of about 50°C to about 70°C, preferably 50°C for a time duration of about 24h for checking the loss of biomass during pre-treatment.

In a non-limiting embodiment, the dried pulp obtained after organic acid treatment is further subjected to inorganic acid treatment using nitric acid (nitration step), wherein the nitric acid and dried pulp are in the concentration ranging from about 0.2:1 to about 1:1.

In another non-limiting embodiment, the inorganic acid treatment step is followed by subjecting the obtained inorganic acid (nitric acid) treated solution to heat treatment at a temperature ranging from about 100°C to about 130°C, preferably about 120°C; for time period ranging from about 20 min to about 40 min, preferably about 30 minutes; cooling the obtained solution to RT; washing and filtering to obtain a pulp, which is further dried to a temperature ranging from about 30°C to about 60°C, preferably about 50°C to obtain pre-treated biomass in the form of dried pulp.

In still another non-limiting embodiment, the organic acid (formic acid) breaks the linkage between cellulose, hemicellulose and lignin; and the inorganic acid (nitric acid) treatment results in oxidation and nitration of lignin. Delignification using nitric acid involves cleavage of β-aryl ether (which are the main intra lignin linkages) in addition to cleavage of α-aryl ether (lignin-carbohydrate, lignin- lignin) and other linkages. This results in disassociation of lignin and carbohydrates (cellulose and hemicellulose) and also breaking of the lignin polymer, thereby making the biomass more amenable to subsequent steps of deconstruction. Therefore, the pre-treatment step in the process of the instant disclosure which is carried out by employing organic acid and/or inorganic acid is critical.

In yet another exemplary embodiment, the deconstruction step of biomass treatment involves steps not limiting to removal of formyl cellulosic esters from the biomass, which would thereby make the cellulose available for microbial fermentation.

In yet another exemplary embodiment, the pulp washing and liquor sugars are analyzed on HPLC and the concentration of inhibitory products- hydroxylmethylfurfural, furfural, and levulinic acid is observed to be NIL

In still another non-limiting embodiment, the de-esterification of the pre-treated biomass is carried out by adding a de-esterification agent.

In still another non-limiting embodiment, the de-esterification agent is an alkali selected from but not limited to group comprising sodium carbonate, sodium hydroxide, potassium hydroxide, calcium hydroxide, calcium carbonate, calcium oxide and ammonia or any combinations thereof at concentration ranging from about 0. 005%w/v to about 1% w/v, preferably about 0.5% w/v. The workable ratio of sodium carbonate (g) : biomass (g) is in the range of about 1:1 to about 1:15.

In still another non-limiting embodiment, the de-esterification step involves the treatment with alkali by heating the biomass to temperature ranging from about 80°C to about 110°C, preferably about 100°C; with constant stirring at about 50 rpm to about 300 rpm, preferably about 200 rpm for time-period ranging from about 5 min to about 30 min, preferably about 10 minutes.

In still another non-limiting embodiment, the combined sequential steps of pre-treatment employing formic acid treatment followed by nitric acid treatment and de-esterification step employing Na2CO3 treatment of woody biomass provide greater sugar yield in the enzymatic hydrolysis step.

In a preferred embodiment, glucose obtained after enzymatic hydrolysis is analyzed by HPLC.

In another preferred embodiment, glucose obtained after enzymatic hydrolysis has a purity of about 81.50%.

In still another non-limiting embodiment, the liquor obtained after inorganic acid treatment is neutralized by an alkali.

In still another non-limiting embodiment, the alkali is selected from but not limited to group comprising sodium carbonate, sodium hydroxide, potassium hydroxide, calcium hydroxide, calcium carbonate, calcium oxide and ammonia or any combinations thereof.

In a preferred embodiment, sodium carbonate is used for neutralization.

In still another non-limiting embodiment, the biomass is subjected to enzymatic treatment post the de-esterification step.

In a preferred embodiment, the dried biomass pulp obtained after the de-esterification step is subjected to enzymatic treatment.

In another exemplary embodiment, the present disclosure provides a method for treatment of biomass in order to render the biomass more amenable to enzymatic hydrolysis.

In yet another exemplary embodiment, the biomass pulp de-esterification is carried out by employing a de-esterification agent, not limiting to sodium carbonate (de-esterifying agent). Sodium carbonate improves amenability of the biomass, not limiting to recalcitrant biomass such as cotton stalks, to enzymatic hydrolysis, as sodium carbonate assists in removing the formic acid (organic acid) from cellulose formate (present in biomass pulp). Employing sodium carbonate thereby helps in increasing hydrolysis of the de-esterified pulp.

The present disclosure provides a method for conversion of recalcitrant biomass, wherein the method comprises use of a weak base, such as but not limited to sodium carbonate as de-esterification agent for removal of formate from cellulose formate produced during the formic acid pre-treatment of the biomass such as but not limited to cotton, castor and red gram biomass.

In an embodiment, using weak base helps in downstream process, such as neutralization will need less acid.

In a non-limiting embodiment, the method for treatment of biomass with organic acid including step of de-esterifying the biomass improves the final recovery of sugars from biomass by atleast 30%.

In a non-limiting embodiment, the method for treatment of biomass with organic acid, inorganic acid and including step of de-esterifying the biomass improves the final recovery of sugars from biomass preferably by at least 50%.

In another non-limiting embodiment, the method of pre-treating biomass provided by the present disclosure not only improves the quality of biomass as a substrate for enzyme hydrolysis in a much shorter time but also ensures that the hydrolysis can be completed with less enzyme load.

In yet another exemplary embodiment, the hydrolysis step of biomass treatment involves step not limiting to enzymatic hydrolysis of the biomass.

In yet another non-limiting embodiment, the enzyme hydrolysis step of the instant disclosure includes subjecting the de-esterified pulp to the action of enzymes, which is a mixture of cellulose, xylanase and beta glucosidase .

In still another non-limiting embodiment, the enzyme hydrolysis step employs cellulase enzymes selected from a group comprising, but not restricting to Novozymes Cellic Ctec 3, Sacchariseb C6 (Advanced enzymes), Codexyme (Codexis), Duet (Genencor) and Cocktail (Dyadic).

In a preferred embodiment, the enzyme hydrolysis step of the instant disclosure is carried out by employing Novozymes Cellic Ctec 3.

In still another non-limiting embodiment, the enzymes employed in the enzyme hydrolysis reaction are workable at concentration ranging from about 0.05FPU/g to about 100FPU/g, preferably at about 25FPU/g.

In still another non-limiting embodiment, the enzyme hydrolysis step is carried out at pH ranging from about 4 to about 5, preferably at about 4.8; incubation temperature ranging from about 40°C to about 60°C, preferably at about 50°C; and with agitation speed ranging from about 150rpm to about 250 rpm, preferably at about 200rpm.

In still another non-limiting embodiment, the method of the present disclosure provides to utilize cotton stalk, castor stalk, red gram stalk, sesame stalk, sugarcane top, blackgram stalk, wheat straw and groundnut stalk as a feedstock wherein optimal pre-treatment is mandated to render the cellulose present in said feedstocks, more amenable to the action of hydrolytic enzymes as well as ensure that the hydrolysate obtained has minimal inhibitors so that it can be used for fermentation.

The present disclosure also determines the effect of formic acid and nitric acid on pre-treatment of cotton stalks, castor stalks, red gram stalks, sesame stalk, sugarcane top, blackgram stalk, wheat straw and groundnut stalk; and also presents de-esterification reaction by employing de-esterification agent such as but not limiting to sodium carbonate, whereby the reaction of such agents with the pre-treated pulp results in high glucose yield from the pulp after enzyme hydrolysis.

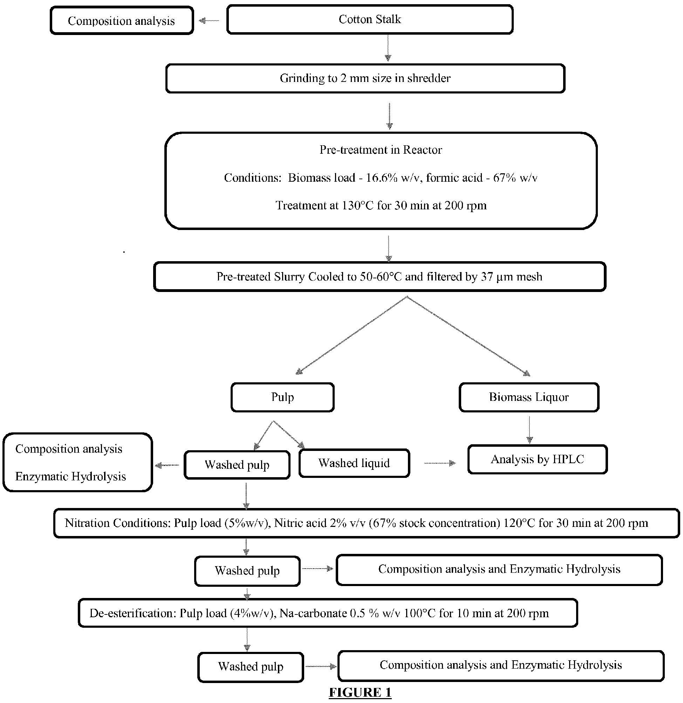

In an exemplary embodiment, the flow chart of the process of biomass treatment of the instant disclosure is illustrated as per Figure 1.

Some of the advantages offered by the method of the present disclosure are as follows:
- Low cost process for pre-treatment of biomass, such as recalcitrant biomass; as cheap alkali like sodium carbonate is used as the de-esterification agent making the process economical and more efficient.
- The process of the present disclosure is faster and more efficient, as less time is required for hydrolysis of pre-treated and de-esterified pulp.
- The process employed in the present disclosure is eco-friendly as it does not release any waste from process to environment.
- The overall process includes de-esterification of pre-treated pulp using de-esterifying agents not limiting to sodium carbonate for maximum recovery of sugars from recalcitrant biomass such as, but not limiting to cotton stalks.
- Improved enzymatic hydrolysis of pre-treated and de-esterified pulp with respect to recovery of sugars.
- Less enzyme loading for hydrolysis of pre-treated and de-esterified pulp.
- Fraction of C5 sugars containing significantly less amount of lignin monomers are obtained in this process.

In a non-limiting embodiment of the present disclosure, Novozymes Cellic Ctec 3 is a formulation of a mixture comprising cellulase, xylanase and beta glucosidase enzymes and both the terms have the same meaning and scope within the ambit of the disclosure.

The method of the present disclosure provides for a low-cost alternative for application as a biofuel in automobiles.

The present disclosure herein below provides for certain examples for better understanding of the instant disclosure. For the purpose of illustration, a method for pre-treatment of recalcitrant biological material; wherein the biological material is biomass and more preferably lignocellulosic biomass is provided. However, person skilled in the art would be aware that the illustrations provided herein are only prospective in nature and can be extrapolated to any type of biomass including but not limiting to lignocellulosic biomass which further includes but are not limited to cotton stalk, castor stalk, red gram, sesame stalk, sugarcane top, blackgram stalk, wheat straw, groundnut stalk etc. with modifications which fall under the purview of the instant disclosure.

Additional embodiments and features of the present disclosure will be apparent to one of ordinary skill in art based upon description provided herein. The embodiments herein provide various features and advantageous details thereof in the description. Descriptions of well-known/conventional methods and techniques are omitted to not unnecessarily obscure the embodiments herein. Further, the disclosure herein provides for examples illustrating the above described embodiments, and in order to illustrate the embodiments of the present disclosure certain aspects have been employed. The examples used herein are intended merely to facilitate an understanding of ways in which the embodiments herein may be practiced and to further enable those of skill in the art to practice the embodiments herein.

Unless the context clearly requires otherwise, throughout the description and the claims, the words "comprise", "comprising", and the like are to be construed in an inclusive sense as opposed to an exclusive or exhaustive sense; that is to say, in the sense of "including, but not limited to".

Although the disclosure will be described with reference to specific examples it will be appreciated by those skilled in the art that the disclosure may be embodied in many other forms.

Accordingly, the following examples should not be construed as limiting the scope of the embodiments herein.

### EXAMPLES

In the present disclosure, the biomass (biological material) employed have the following source and geographical origin:
- cotton stalk - Jaitapur, Rajkot district of Rajasthan.

### Example 1: Pre-treatment, de-esterification and enzymatic hydrolysis of cotton stalk.

### Pre-treatment

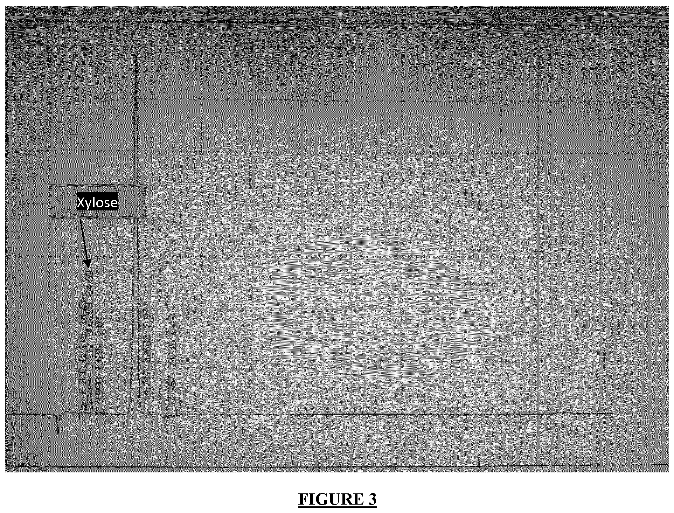

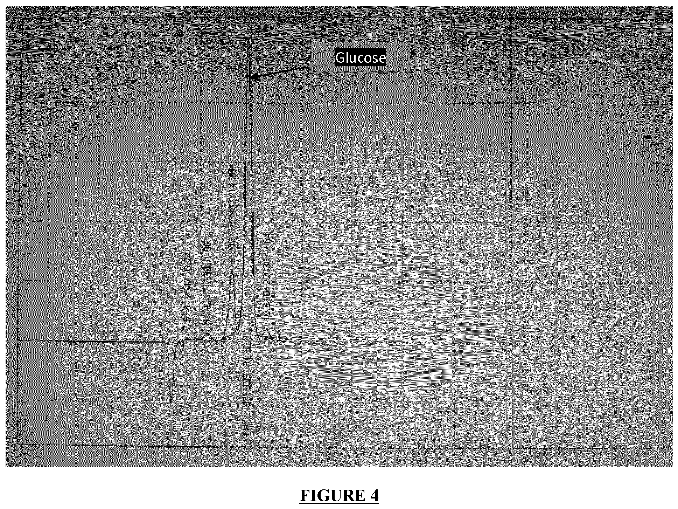

Cotton stalk is subjected to grinding to about 2mm to about 5mm size in a shredder or pre-shredded cotton stalk of 2mm-5mm size is taken. Moisture content of crushed cotton biomass is checked by moisture analyzer and all the experiments herein are carried out on moisture-free and dry weight basis. The crushed biomass is subjected to mild organic acid pre-treatment in reactor. This pre-treatment involves a biomass load of about 16.6% w/v and formic acid concentration of about 67% w/v with solid to solvent ratio of about 1:4 in a total volume of about 200 ml. Reaction conditions are about 130°C for about 30 min with about 200 rpm agitation speed. After organic acid pre-treatment, the whole slurry from the reactor is cooled to about 50-60°C and filtered through 297 µm mesh followed by 37 µm mesh, to obtain pulp and liquor. The liquor passing through the mesh is collected and stored at about 4°C for further analysis. The pulp is subjected to tap water wash (2L) to remove the attached formic acid, xylose and other components. The 2L wash (or washing/liquid obtained post washing of the pulp) is also kept at about 4°C for further analysis on HPLC. The washed pulp is dried at about 50°C and weighed to check the mass loss after pre-treatment. Moisture content of pulp is checked by moisture analyzer. The pulp is subjected to composition analysis for determination of hexosan, pentosan and acid insoluble lignin content as per NREL protocol. The sugar (glucose, xylose, arabinose) content of pulp washing and liquor is analyzed on HPLC and the concentration of inhibitory products- hydroxylmethylfurfural, furfural, and levulinic acid is observed to be NIL (Figures 3 and 4).

NREL protocol employed in the instant disclosure involves about 300 mg of biomass (Cotton stalk, Castor stalk, Red gram biomass, sesame stalk, sugarcane top, blackgram stalk, groundnut stalk, wheat straw or combinations thereof) sample is weighed and about 3.00 ml of 72% sulphuric acid is added. The sample is then placed in a water bath set at about 30°C and incubated for about 60 minutes. Upon completion of the hydrolysis, samples are removed from the water bath and the acid in the sample is diluted to about 4% concentration by adding about 84.00 mL deionized water. After dilution, the samples are autoclaved for about 60 minutes, filtered and neutralized. The filtered sample is finally analyzed on HPLC using HPX87H column with RID (Refractive Index Detector) detector. The mobile phase for HPLC is 0.05M sulphuric acid and the flow rate maintained is 0.6ml/min.

### De-esterification of acid (organic acid) pre-treated biomass:

The organic acid pre-treated and dried pulp is subjected to de-esterification using sodium carbonate. The pre-treated pulp (4 gm), is added to about 100 ml tap water and pH of the solution is raised to about 11 using 0.5 g solid sodium carbonate. The solution thereafter is subjected to heating on a hot plate at temperature of about 100°C with stirring at about 200 rpm for about 10 min. The solution is cooled to RT and obtained pulp is washed with tap water using 37µm mesh filter until the pH is neutral. The de-esterified pulp is dried at about 50°C and the moisture content is checked by moisture analyzer. The de-esterified and dried pulp is subjected to composition analysis as per NREL protocol.

### Enzymatic Hydrolysis of acid (organic acid) pre-treated cotton pulp and de-esterified cotton pulp.

The pulp obtained after formic acid pre-treatment and de-esterification are subjected to enzymatic hydrolysis using Novozymes Cellic Ctec3. Enzyme loading is varied (25- 100 FPU) and sugar released is analyzed at various time intervals (24h, 48h, 120h). Conditions of enzymatic hydrolysis are: pulp load 5% w/v, sodium citrate buffer pH 4.8 (50 mM), incubation temperature about 50°C, and agitation about 200 rpm. Mass balance of cotton stalk pulp at various stages of pre-treatment is given below.

**Table 1: Mass Balance after formic acid pre-treatment**

| **Cellulose Mass Balance** | | |
|---|---|---|
| Initial Biomass g | % of cellulose content | Cellulose in g |
| 33.33 | 35.18 | 11.73 |
| Pre-treated Biomass g | % of cellulose content | Cellulose in g |
| 19.46 | 49.70 | 9.67 |
| | **Cellulose recovery %** | **82**.**48** |

| **Hemicellulose Mass Balance** | | |
|---|---|---|
| Initial Biomass g | % of Hemicellulose content | Hemicellulose in g |
| 33.33 | 14.69 | 4.90 |
| Pre-treated Biomass g | % of Hemicellulose content | Hemicellulose in g |
| 19.46 | 4.66 | 0.91 |
| | **Hemicellulose recovery %** | **18.52** |

| **Lignin Mass Balance** | | |
|---|---|---|
| % Lignin + Ash in pulp | 35.42 | |
| % of ash | 0.15 | |
| % of lignin | 35.27 | |
| Initial Biomass g | % of Lignin content | Lignin in g |
| 33.33 | 24.20 | 8.07 |
| Pre-treated Biomass g | % of Lignin content | Lignin in g |
| 19.46 | 35.27 | 6.86 |
| | **Lignin recovery %** | **85.09** |

Inference: Cellulose and hemicellulose recovery after formic acid pre-treatment is 82.48% and 18.52% respectively; lignin recovery is 85.09% and delignification is 14.91%.

**Table 2: Mass Balance after formic acid pre-treatment and de-esterification**

| **Cellulose Mass Balance** | | |
|---|---|---|
| Initial Biomass g | % of cellulose content | Cellulose in g |
| 33.33 | 35.18 | 11.73 |
| Pre-treated Biomass g | % of cellulose content | Cellulose in g |
| 16.56 | 57.22 | 9.48 |
| | **Cellulose recovery %** | **80.81** |

| **Hemicellulose Mass Balance** | | |
|---|---|---|
| Initial Biomass g | % of Hemicellulose content | Hemicellulose in g |
| 33.33 | 14.69 | 4.90 |
| Pre-treated Biomass g | % of Hemicellulose content | Hemicellulose in g |
| 16.56 | 3.94 | 0.65 |
| | **Hemicellulose recovery %** | **13.33** |

| **Lignin Mass Balance** | | |
|---|---|---|
| % Lignin + Ash in pulp | 33.47 | |
| % of ash | 0.16 | |
| % of lignin | 33.31 | |
| Initial Biomass g | % of Lignin content | Lignin in g |
| 33.33 | 24.20 | 8.07 |
| Pre-treated Biomass g | % of Lignin content | Lignin in g |
| 16.56 | 33.31 | 5.52 |
| | **Lignin recovery %** | **68.39** |

Inference: Cellulose and hemicellulose recovery after formic acid pre-treatment and de-esterification is 80.81% and 13.33% respectively; lignin recovery is 68.39% and delignification is 31.61 %.

### Glucose g/100gm of cotton stalk (% cellulose hydrolysis)

**Table 3: Enzymatic Hydrolysis of Formic acid treated pulp at various enzyme loadings**

| Hrs | Formic acid treated Pulp | | | |
|---|---|---|---|---|
| | E-25FPU/gm | E-50FPU/gm | E-75FPU/gm | E-100FPU/gm |
| 24 | 6.07(21.55 %) | 8.45(30%) | 9.65(34.28%) | 10.23(36.21%) |
| 48 | 7.25(25.73%) | 10.27(36.45%) | 11.65(41.36%) | 13.22(46.94%) |
| 120 | 8.77(31.13%) | 12.34(43.83%) | 15.59(55.34%) | 16.62(59.02%) |

### Glucose g /100gm of cotton stalk (% cellulose hydrolysis)

**Table 4: Enzymatic Hydrolysis of De-esterified pulp at various enzyme loadings**

| Hrs | De-esterified Pulp | | | |
|---|---|---|---|---|
| | E-25FPU/gm | E-50FPU/gm | E-75FPU/gm | E-100FPU/gm |
| 24 | 11.77(45.88%) | 14.64(57.09%) | 15.99(62.99%) | 17.42(67.93%) |
| 48 | 12.73(49.64%) | 17.41(67.89%) | 18.05(70.38%) | 19.46(75.87%) |
| 120 | 13.43(52.36%) | 17.82(69.46%) | 20.89(81.44%) | 22.95(89.49%) |

Inference: Glucose released after de-esterification is 22.95 g/100g of cotton stalk with 100FPU Cellic Ctec 3 while after formic acid pre-treatment 16.62 g of glucose is released.

### Over all mass balance:

**Table 5: Glucose mass balance: All Fractions**

| **Glucose from various fractions** | **in g** |
|---|---|
| Initial Glucose in Biomass | 39.08 |
| Glucose from pulp after enzymatic hydrolysis | 22.95 |
| Glucose from biomass liquor (which includes washing) | 2.73 |
| **Over all glucose in g /100g Biomass** | **25.68** |

**Table 6: Xylose mass balance: All Fractions**

| **Xylose from various fractions** | **in g** |
|---|---|
| Initial Xylose in Biomass | 16.69 |
| Xylose from biomass liquor (which includes washing) | 10.26 |
| **Over all xylose in g /100g Biomass** | **10.26** |

### 100FPU enzyme load

**Table 7: Over all Yield per 100gm of Biomass**

| | | |
|---|---|---|
| Initial Cellulose g | 35.18 | **Hexose yield % 72.99** |
| Glucose in g | 25.68 | |
| Initial Hemicellulose g | 14.69 | **Pentose yield % 69.84** |
| Xylose in g | 10.26 | |

### Example 2: Pre-treatment (organic acid followed by inorganic acid), de-esterification and enzymatic hydrolysis of cotton stalk.

### Pre-treatment of biomass using organic acid treatment:

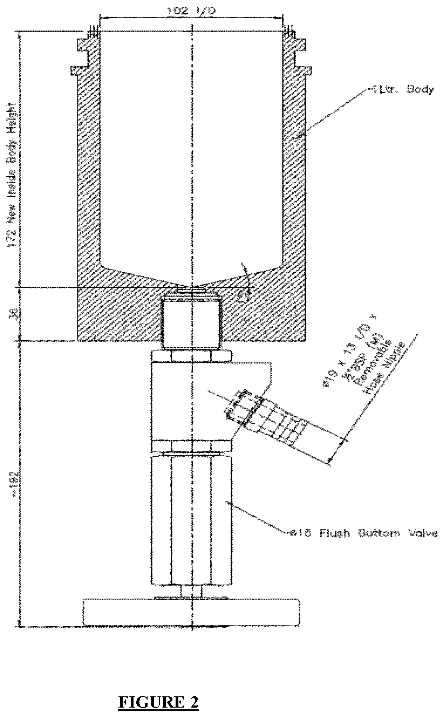

The biomass- cotton stalk is subjected to grinding to about 2mm to about 5mm size in a shredder or pre-shredded cotton stalk of 2mm-5mm size is taken. Moisture content of crushed cotton biomass is checked by moisture analyzer. The crushed biomass is subjected to mild organic acid pre-treatment in a stirred-tank reactor (Figure 2). The biomass load employed is at concentration of 16.6% w/v and formic acid is at concentration of 67% w/v, in a total volume of about 400 ml. Reactor conditions are about 130°C for about 30 min with about 200 rpm agitation speed. After organic acid pre-treatment, the whole slurry from the reactor is cooled to about 50-60°C and filtered through about 297 µm mesh followed by about 37 µm mesh, to obtain pulp and liquor. The liquor passing through the mesh is collected and kept at about 4°C for further analysis. The pulp is subjected to tap water wash (2L) to remove the attached formic acid, xylose and other components. The 2L wash is also kept at about 4°C for further analysis on HPLC. The washed pulp is dried at about 50°C and weighed to check the mass loss after pre-treatment. Moisture content of pulp is checked by moisture analyzer. The pulp is subjected to composition analysis for determination of hexosan, pentosan and acid insoluble lignin content as per NREL protocol. The sugar (glucose, xylose, arabinose) content of pulp washing and liquor is analyzed on HPLC and the concentration of inhibitory products-hydroxylmethylfurfural, furfural, and levulinic acid is observed to be NIL (Figures 3 and 4).

### Inorganic acid treatment (Nitration) of orgnic acid pre-treated biomass

The organic acid pre-treated and dried pulp is subjected to a step of inorganic acid treatment or nitration using about 1% (w/w) and about 2% (w/w) nitric acid. The organic acid pre-treated pulp (5 gm), is added to about 100 ml tap water containing about 1% (w/w) and about 2% (w/w)nitric acid. Obtained solution is heated to temperature of about 120°C for about 30 min in the reactor. The solution thereafter is cooled to RT and washed with tap water using about 37µm mesh filter until the pH became neutral. The solution comprising the pulp is dried at about 50°C to obtain a pre-treated biomass and the moisture content is checked by moisture analyzer. The dried pulp is subjected to composition analysis as per NREL protocol.

### De-esterification of pre-treated biomass

After inorganic acid treatment, the dried pulp (pre-treated biomass/pulp) is subjected to de-esterification using sodium carbonate. The pre-treated pulp (4 gm), is added to about 100 ml tap water and pH of the solution is raised to about 11 using 0.5 g solid sodium carbonate. The solution thereafter is subjected to heating on a hot plate at temperature of about 100°C with stirring at about 200 rpm for about 10 min. The solution is cooled to RT and obtained pulp is washed with tap water using about 37µm mesh filter until the pH is neutral. The de-esterified pulp is dried at about 50°C and the moisture content is checked by moisture analyzer. The de-esterified and dried pulp is subjected to composition analysis as per NREL protocol.

### Enzymatic Hydrolysis of formic acid pre-treated cotton pulp, nitric acid pre-treated pulp and de-esterified cotton pulp.

The pulp obtained after formic acid pre-treatment, nitric acid pre-treatment and de-esterification are subjected to enzymatic hydrolysis using Novozymes Cellic Ctec3. Enzyme loading is varied (from about 10 FPU to about 25 FPU) and sugar released is analyzed by HPLC at various time intervals (48h) (Figure 4). Conditions of enzymatic hydrolysis are: pulp load 5% w/v, sodium citrate buffer pH 4.8 (50 mM), incubation temperature about 50°C and agitation about 200 rpm.

Mass balance of cotton stalk pulp is given below:

**Table 8: Mass Balance after formic acid pre-treatment**

| **Cellulose Mass Balance** | | |
|---|---|---|
| Initial Biomass g | % of cellulose content | Cellulose in g |
| 66.66 | 32.18 | 21.45 |
| Pre-treated Biomass g | % of cellulose content | Cellulose in g |
| 42.03 | 42.25 | 17.76 |
| | **Cellulose recovery %** | **82.78** |

| **Hemicellulose Mass Balance** | | |
|---|---|---|
| Initial Biomass g | % of Hemicellulose content | Hemicellulose in g |
| 66.66 | 12.86 | 8.57 |
| Pre-treated Biomass g | % of Hemicellulose content | Hemicellulose in g |
| 42.03 | 4.34 | 1.82 |
| | **Hemicellulose recovery %** | **21.28** |

| **Lignin Mass Balance** | | |
|---|---|---|
| % Lignin + Ash in pulp | 34.38 | |
| % of ash | 0.17 | |
| % of lignin | 34.21 | |
| Initial Biomass g | % of Lignin content | Lignin in g |
| 66.66 | 24.20 | 16.13 |
| Pretreated Biomass g | % of Lignin content | Lignin in g |
| 42.03 | 34.21 | 14.38 |
| | **Lignin Recovery %** | **89.13** |

Inference: The amount of cellulose and hemicellulose recovery after formic acid pre-treatment is 82.78% and 21.28% respectively. Lignin recovery is 89.13%, delignification is 10.87%.

Mass Balance after formic acid pre-treatment and nitration with 1% (w/w) nitric acid

**Table 9: Mass Balance after formic acid pre-treatment and nitration with 1 % (w/w) nitric acid**

| **Cellulose Mass Balance** | | |
|---|---|---|
| Initial Biomass g | % of cellulose content | Cellulose in g |
| 66.66 | 32.18 | 21.45 |
| Pretreated Biomass g | % of cellulose content | Cellulose in g |
| 32.46 | 52.92 | 17.18 |
| | **Cellulose recovery %** | **80.08** |

| **Hemicellulose Mass Balance** | | |
|---|---|---|
| Initial Biomass g | % of Hemicellulose content | Hemicellulose in g |
| 66.66 | 12.86 | 8.57 |
| Pretreated Biomass g | % of Hemicellulose content | Hemicellulose in g |
| 32.46 | 2.34 | 0.76 |
| | **Hemicellulose recovery %** | **8.86** |

| **Lignin Mass Balance** | | |
|---|---|---|
| % Lignin + Ash in pulp | 18.20 | |
| % of ash | 0.15 | |
| % of lignin | 18.05 | |
| Initial Biomass g | % of Lignin content | Lignin in g |
| 66.66 | 24.20 | 16.13 |
| Pretreated Biomass g | % of Lignin content | Lignin in g |
| 32.46 | 18.05 | 5.86 |
| | **Lignin Recovery %** | **36.32** |

Inference: The amount of cellulose and hemicellulose recovery after formic acid pre-treatment and nitration with 1 % (w/w) nitric acid are 80.08% and 8.86% respectively. 36.32 % of lignin is present in formic acid pre-treated and nitrated pulp.

Mass Balance after formic acid pre-treatment and nitration with 1% (w/w) nitric acid and de-esterification with 0.5% w/v Na-carbonate

**Table 10: Mass Balance after formic acid pre-treatment, nitration with 1 % w/w nitric acid and de-esterification with 0.5% w/v Na-carbonate.**

| **Cellulose Mass Balance** | | |
|---|---|---|
| Initial Biomass g | % of cellulose content | Cellulose in g |
| 66.66 | 32.18 | 21.45 |
| Pretreated Biomass g | % of cellulose content | Cellulose in g |
| 29.68 | 55.05 | 16.34 |
| | **Cellulose recovery %** | **76.17** |

| **Hemicellulose Mass Balance** | | |
|---|---|---|
| Initial Biomass g | % of Hemicellulose content | Hemicellulose in g |
| 66.66 | 12.86 | 8.57 |
| Pretreated Biomass g | % of Hemicellulose content | Hemicellulose in g |
| 29.68 | 2.37 | 0.70 |
| | **Hemicellulose recovery %** | **8.21** |

| **Lignin Mass Balance** | | |
|---|---|---|
| % Lignin + Ash in pulp | 16.80 | |
| % of ash | 0.20 | |
| % of lignin | 16.60 | |
| Initial Biomass g | % of Lignin content | Lignin in g |
| 66.66 | 24.20 | 16.13 |
| Pretreated Biomass g | % of Lignin content | Lignin in g |
| 29.68 | 16.60 | 4.93 |
| | **Lignin Recovery %** | **30.54** |

Inference: The amount of lignin in pulp is 30.54 % w/w after formic acid pre-treatment, nitration and de-esterification.

Conclusion: 76.17 % of cellulose and 8.21% of hemicellulose is retained in pulp after formic acid pre-treatment, nitration with 1 % (w/w) nitric acid and de-esterification with 0.5% w/v Na-carbonate.

**Table 11: Mass Balance after formic acid pre-treatment and nitration with 2 % (w/w) nitric acid**

| **Cellulose Mass Balance** | | |
|---|---|---|
| Initial Biomass g | % of cellulose content | Cellulose in g |
| 66.66 | 32.18 | 21.45 |
| Pretreated Biomass g | % of cellulose content | Cellulose in g |
| 26.89 | 59.42 | 15.98 |
| | **Cellulose recovery %** | **74.49** |

| **Hemicellulose Mass Balance** | | |
|---|---|---|
| Initial Biomass g | % of Hemicellulose content | Hemicellulose in g |
| 66.66 | 12.86 | 8.57 |
| Pretreated Biomass g | % of Hemicellulose content | Hemicellulose in g |
| 26.89 | 3.23 | 0.87 |
| | **Hemicellulose recovery %** | **10.13** |

| **Lignin Mass Balance** | | |
|---|---|---|
| % Lignin + Ash in pulp | 13.30 | |
| % of ash | 0.21 | |
| % of lignin | 13.09 | |
| Initial Biomass g | % of Lignin content | Lignin in g |
| 66.66 | 24.20 | 16.13 |
| Pretreated Biomass g | % of Lignin content | Lignin in g |
| 26.89 | 13.09 | 3.52 |
| | **Lignin Recovery %** | **21.82** |

Inference: The amount of cellulose and hemicellulose recovery after formic acid pre-treatment and nitration with 2 % (w/w) nitric acid are 74.49% and 10.13% respectively. 21.82 % w/w of lignin is present in formic acid pre-treatment and nitration of pulp.

**Table 12: Mass Balance after formic acid pre-treatment, nitration with 2 % (w/w) nitric acid and de-esterification with 0.5% w/v Na-carbonate.**

| **Cellulose Mass Balance** | | |
|---|---|---|
| Initial Biomass g | % of cellulose content | Cellulose in g |
| 66.66 | 32.18 | 21.45 |
| Pretreated Biomass g | % of cellulose content | Cellulose in g |
| 21.92 | 71.64 | 15.70 |
| | **Cellulose recovery %** | **73.21** |

| **Hemicellulose Mass Balance** | | |
|---|---|---|
| Initial Biomass g | % of Hemicellulose content | Hemicellulose in g |
| 66.66 | 12.86 | 8.57 |
| Pretreated Biomass g | % of Hemicellulose content | Hemicellulose in g |
| 21.92 | 2.78 | 0.61 |
| | **Hemicellulose recovery %** | **7.11** |

| **Lignin Mass Balance** | | |
|---|---|---|
| % Lignin + Ash in pulp | 12.66 | |
| % of ash | 0.15 | |
| % of lignin | 12.51 | |
| Initial Biomass g | % of Lignin content | Lignin in g |
| 66.66 | 24.20 | 16.13 |

| Pretreated Biomass g | % of Lignin content | Lignin in g |
|---|---|---|
| 21.92 | 12.51 | 2.74 |
| | **Lignin Recovery %** | **17.00** |

Inference: The amount of lignin in pulp is 17.00 % w/w after formic acid pre-treatment, nitration and de-esterification.

Conclusion: 73.21 % of cellulose and 7.11% of hemicellulose is retained in pulp after formic acid pre-treatment, nitration with 2 % (w/w) nitric acid and de-esterification with 0.5% w/v Na-carbonate.

### g of glucose / 100 of biomass (% of hydrolysis of pulp)

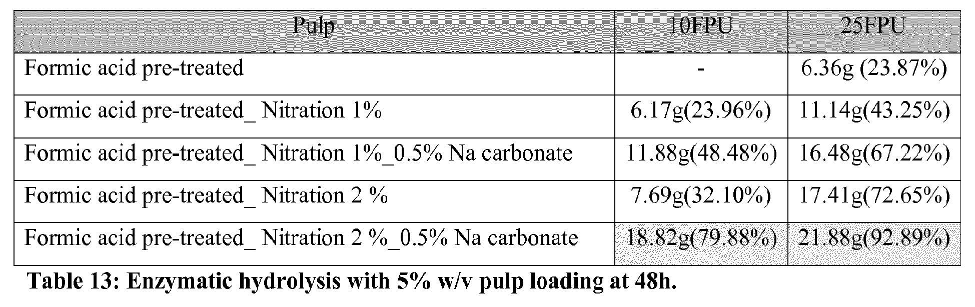

Conclusion: 21.88 g glucose (92.89% pulp hydrolysis) is observed with 25FPU/g pulp.

### 10FPU enzyme load

| Over all Yield per 100gm of Biomass | | |
|---|---|---|
| Initial Cellulose g | 32.18 | **Glucose yield % 68.46** |
| Glucose yield in g | 22.03 | |
| Initial Hemicellulose g | 12.86 | **Pentose yield % 61.98** |
| Pentose yield in g | 7.97 | |

### 25FPU enzyme load

**Table 14: Over all Mass Balance with 10FPU and 25 FPU enzyme load.**

| Over all Yield per 100gm of Biomass | | |
|---|---|---|
| Initial Cellulose g | 32.18 | **Glucose yield % 77.97** |
| Glucose yield in g | 25.09 | |
| Initial Hemicellulose g | 12.86 | **Pentose yield % 61.98** |
| Pentose yield in g | 7.97 | |

Inference: 68.46% and 61.98% of glucose and pentose yield is observed at 10FPU enzyme load and a higher yield of glucose 77.97% is observed at 25FPU enzyme load.

The foregoing description of the specific embodiments will so fully reveal the general nature of the embodiments herein that others can, by applying current knowledge, readily modify and/or adapt for various applications such specific embodiments without departing from the generic concept, and, therefore, such adaptations and modifications should and are intended to be comprehended within the meaning and range of equivalents of the disclosed embodiments. It is to be understood that the phraseology or terminology employed herein is for the purpose of description and not of limitation. Therefore, while the embodiments in this disclosure have been described in terms of preferred embodiments, those skilled in the art will recognize that the embodiments herein can be practiced with modification within the spirit and scope of the embodiments as described herein.

## Claims

1. A process for conversion of biomass to organic compound(s) comprising acts of:
pre-treating the biomass with organic acid or inorganic acid or both;
subjecting the pre-treated biomass to de-esterification; and
subjecting the de-esterified biomass to enzyme hydrolysis, thereby converting the biomass to the organic compound(s).

2. The process as claimed in claim 1, wherein the biomass is selected from a group comprising red gram stalk, cotton stalk, castor stalk, black gram stalk, sugarcane top, sesame stalk, wheat straw and groundnut stalk or any combination thereof; wherein said biomass comprises complex carbohydrates

3. The process as claimed in claim 1, wherein the organic compound(s) is sugar(s), which is selected from a group comprising glucose, xylose, hexose, pentose and arabinose or any combination thereof.

4. The process as claimed in claim 1, wherein the biomass is subjected to shredding or grinding before pre-treatment; and wherein the biomass is in concentration ranging from about 10% w/v to about 20% w/v.

5. The process as claimed in claim 1, wherein the pre-treating comprises the biomass to be subjected to a step of organic acid treatment, followed by inorganic acid treatment; and wherein the process enhances the conversion of biomass to sugar(s) by at least 50%, when compared to a process devoid of pre-treatment with inorganic acid.

6. The process as claimed in claim 1, wherein the organic acid pre-treatment comprises contacting the biomass with organic acid selected from a group comprising formic acid, acetic acid or a combination thereof; and wherein the organic acid is, in concentration ranging from about 50% w/v to about 90% w/v.

7. The process as claimed in claim 6, wherein the organic acid treated biomass is further subjected to washing and filtering to obtain organic acid pre-treated biomass in the form of pulp and liquor; and wherein the pulp obtained is further subjected to wash treatment followed by drying.

8. The process as claimed in claim 5, wherein the inorganic acid pre-treatment comprises contacting the biomass pulp obtained after the organic acid treatment with the inorganic acid selected from a group comprising nitric acid, sulphuric acid and hydrochloric acid or any combination thereof; and wherein the inorganic acid in concentration ranging from about 0.1% (w/w) to about 5% (w/w).

9. The process as claimed in claim 8, wherein the inorganic acid treated biomass is further subjected to washing and filtering to obtain pre-treated biomass in the form of pulp and liquor; and wherein the pulp obtained is further subjected to wash treatment followed by drying.

10. The process as claimed in claim 1, wherein the pre-treatment is carried out in a reactor, at a temperature ranging from about 100°C to about 180°C for a time-period ranging from about 10 min to about 60 minutes with agitation speed of about 50 rpm to about 500 rpm.

11. The process as claimed in any of claims 1-10, wherein ratio of the organic acid to the biomass ranges from about 4:1 to about 10:1, and wherein ratio of the inorganic acid to the biomass ranges from about 0.2:1 to about 1:1.

12. The process as claimed in claim 1, wherein the de-esterification is carried out by subjecting the pre-treated biomass pulp to alkali treatment, wherein the alkali is selected from a group comprising sodium carbonate, sodium hydroxide, potassium hydroxide, calcium hydroxide, calcium carbonate, calcium oxide and ammonia or any combination thereof; which is in concentration ranging from about 0.005% (w/v) to about 1% (w/v)and wherein the ratio of alkali: biomass ranges from about 1:1 to about 1:15 or the alkali is added to the pre-treated biomass pulp until a pH of 11 is obtained.

13. The process as claimed in claim 1, wherein the enzyme hydrolysis is carried out by subjecting the de-esterified biomass to enzyme treatment, wherein the enzyme is a mixture of cellulase, xylanase and beta-glucosidase at a concentration ranging from about 0.05FPU/gram to about 100FPU/gram of biomass.

14. The process as claimed in claim 13, wherein the enzyme hydrolysis is carried out at a pH ranging from about 4 to about 5; incubation temperature ranging from about 40°C to about 60°C; agitation speed ranging from 150 rpm to 250 rpm; and enzyme load of about 25FPU/g to about 100 FPU/g for about 24 hours to about 48 hours.
